# EUROPÄISCHE PATENTSCHRIFT

(11) **EP 1 230 015 B1**
(45) Veröffentlichungstag und Bekanntmachung des Hinweises auf die Patenterteilung: **26.07.2006**
(21) Anmeldenummer: 00985057.9
(22) Anmeldetag: 17.11.2000
(51) Int. Cl.: B01J 19/00, C07B 61/00, C07K 1/04, C07H 21/00

(54) **VERFAHREN ZUR HERSTELLUNG KONFEKTIONIERTER CHEMISCHER OBERFLÄCHEN**
METHOD FOR PRODUCING FINISHED CHEMICAL SURFACES
PROCEDE DE PRODUCTION DE SURFACES CHIMIQUES SURMOULEES

(30) Priorität: 17.11.1999 DE 19955361
(43) Veröffentlichungstag der Anmeldung: 14.08.2002
(73) Patentinhaber: Febit Biotech GmbH, 69120 Heidelberg (DE)
(72) Erfinder: STÄHLER, Peer F., 69167 Mannheim (DE); SCHEFFLER, Matthias, 69493 Hirschberg/Leutershausen (DE); KIESEWETTER, Stefan, 69181 Leimen (DE); GÜIMIL, Ramon, 69126 Heidelberg (DE)
(74) Vertreter: Weiss, Wolfgang
(86) Internationale Anmeldenummer: PCT/EP2000/011476
(87) Internationale Veröffentlichungsnummer: WO 2001/036086

(56) Entgegenhaltungen:
- EP-A- 0 955 085
- WO-A-99/39817
- DE-A- 19 842 164

## Beschreibung

Die Erfindung betrifft ein programmierbares, automatisiertes Verfahren zur Herstellung von konfektionierten chemischen Oberflächen unter kombinatorischer Verwendung spezifischer Synthesemodule durch *in situ* Synthese auf einem Träger. Wichtige Anwendungsgebiete des erfindungsgemäßen Verfahrens sind die molekulare Diagnostik in den Bereichen Human- und Veterinärmedizin (Infektionsdiagnostik, Onkologie, Forensik), die Entwicklung neuer pharmazeutisch wirksamer Substanzen, die biologische Grundlagenforschung, die Lebensmittelanalytik, die Biotechnologie, insbesondere in den Bereichen Stoffproduktion und Umweltanalytik, sowie Hochdurchsatzverfahren zum Wirkstoffscreening in kombinatorisch-chemischen Untersuchungen.

Automatisierte Systeme, die eine schnelle, gezielte und exakte Erfassung und Analyse chemischer und/oder biologischer Informationen erlauben, sind beispielsweise in den Biowissenschaften und angrenzenden Fachgebieten, wie der pharmazeutischen Wirkstoffentwicklung und der medizinischen Diagnostik, unverzichtbare Hilfsmittel.

Ein leistungsfähiges System zur Erfassung und Analyse von großen Informationsmengen, wie sie bei der Erfassung genetischer Informationen oder der Analyse von Produkten der kombinatorischen Chemie anfallen, sind fluidische Mikroprozessoren. Darunter versteht man miniaturisierte, hybride Funktionselemente mit chemischen und technischen Komponenten, z.B. einen Reaktionsträger, an dessen Oberfläche Rezeptoren, z.B. Biomoleküle immobilisiert sind, die als spezifische Interaktionspartner für entsprechende Analyten dienen. Da tausende potentielle biologische und biochemische Interaktionspartner in einem Reaktionsträger angeordnet sein können, müssen diese mit mikrotechnischen Methoden aufgebracht werden.

Nach dem derzeitigem Stand der Technik werden die Rezeptoren durch kovalente oder nicht-kovalente Wechselwirkungen immobilisiert oder in situ auf der Festphase synthetisiert. Eine Modulierung der physikalischen (z.B. Dotierungsdichte), physikalisch-chemischen (z.B. Redoxpotential), chemischen (z.B. katalytische Aktivität) und gegebenenfalls biokompatiblen Eigenschaften (z.B. Steuerung molekularer Erkennungsprozesse) der hergestellten Oberflächen ist experimentell sehr aufwendig und gelingt nicht immer. Optimierungen chemischer, biologischer und biochemischer Interaktionen, die an Oberflächen nachgewiesen werden sollen, sind daher nur unter erheblichem Arbeits- und Zeitaufwand, wenn überhaupt, zu erreichen. Da als Interaktionspartner Naturstoffe wie DNA, RNA, PNA, Oligonukleotide, Saccharide, Kohlenhydrate, Peptide, Proteine, aber auch Derivate der kombinatorischen Chemie in Frage kommen, ist die Bandbreite optimal geeigneter Oberflächenkonfigurationen sehr groß und bislang weder analytisch, noch synthetisch ausreichend systematisch erfaßt.

Die Bausteine eines chemischen Polymerisationsprozesses werden generell als "Synthone" bezeichnet. Die funktionellen Gruppen eines Synthons erlauben eine zielgerichtete chemische Reaktion mit geeigneten anderen funktionellen Gruppen an einem zweiten Synthon. In aller Regel sind diese reaktiven Zentren durch sogenannte Schutzgruppen maskiert, die in einer geeigneten chemischen Umgebung gezielt entfernt werden können, wodurch eine Steuerung des Syntheseprozesses möglich wird, da nur solche funktionellen Gruppen umgesetzt werden, die keine Schutzgruppe tragen. Abhängig von der jeweiligen Synthesestrategie können Schutzgruppen im allgemeinen durch eine Änderung chemischer oder physikochemischer Umgebungsparameter wie dem Redoxpotential, dem pH-Wert oder der Temperatur, aber auch durch den Eintrag elektromagnetischer Energie, z.B. durch Bestrahlung mit Licht einer bestimmten Wellenlänge, entfernt werden.

Für die sequenzspezifische Synthese von Oligomeren, wie Oligonukleotiden, Oligonukleotidderivaten, Peptiden, oder Kohlenhydraten, die aus unterschiedlichen, jedoch endlich vielen Monomereinheiten aufgebaut sind, hat sich die von Merrifield eingeführte, sequentielle Polymerisation durch Kondensation an einer festen Phase bewährt (R.B. Merrifield (1963), J. Am. Chem. Soc. 85: 2149-2154; R.B. Merrifield (1965) Science 150: 179-185). Die Synthese nach Merrifield beginnt an einem trägergebundenen Startmonomer, das durch die Entfernung einer oder mehrerer Schutzgruppen aktiviert werden kann. Dadurch wird die Addition eines in Lösung zugeführten, nächsten Monomers gestattet, das nach erfolgter Polymerisation durch Kondensation seinerseits als Ausgangspunkt für einen weiteren Polymerisationsschritt zur Verfügung steht. Die sukzessive Wiederholung von Aktivierung und nachfolgender Polymerisation durch Kondensation führt schließlich zur Synthese des gewünschten Oligomeren.

Als Trägermaterialien für Merrifield-Synthesen haben sich insbesondere Glasoberflächen bewährt. Aber auch Silizium oder Metalle wie Gold, hochvernetztes Polystyrol und andere Kunststoffe wie leitfähiges Polypyrrol oder Copolymere wie N-Vinylpyrrolidon/N-Acryloxysuccinimid, sowie "Naturstoffe" wie Papier (Cellulose) werden als Trägerstoffe verwendet. Die Funktionalisierung der genannten Materialien erfolgt in der Regel nach dem Fachmann bekannten, chemischen (z.B. Hydrierung mit nachfolgender Substitution) (Immobilized Affinity Ligand Techniques, Academic Press 1992, eds. G.T. Hermason, A.K. Mallia, P.K. Smith) oder physikalischen Standardverfahren (z.B. Plasmabeschichtung), bei denen das Ergebnis der Funktionalisierungsreaktion empirisch eingestellt und überprüft wird. Der Bereich zwischen den funktionellen Gruppen auf der Oberfläche des Trägers und den eigentlichen, bindungsaktiven funktionellen Rezeptoren (z.B. immobilisierte Biomoleküle) wird von sogenannten "Linkern" überbrückt. Dabei handelt es sich um beliebig modifizierte Molekülreste, die funktionelle Gruppen enthalten und über diese Gruppen Bindung von Rezeptoren (z.B. Biomoleküle wie Oligonukleotide oder Kohlenhydratreste) mit der Trägeroberfläche vermitteln. Die physiko-chemischen und die sterisch-räumlichen Eigenschaften des Linkers beeinflussen sowohl die an ihnen ausgeführten Merrifield-Synthesen (J. Katzhendler, S. Cohen, E. Rahmim, M. Weisz, I. Ringel, J. Deutsch (1989) Tetrahedron 45: 2777-2792; A.v. Aerschot, P. Herdewijn, H. Vanderhaeghe (1988), Nucl. Nucl. 7: 75-90), als auch die Bindung des potentiellen Analyten an die hergestellte bindungsaktive Oberfläche (M. Beier, J.D. Hoheisel (1999) Nucleic Acids Res. 27: 1970-1977). Die Auswahl und Varianz der verwendeten Linkerbausteine basiert in der Regel auf Erfahrungswerten und muß empirisch verifiziert werden.

Für eine Reihe trägergebundener, nukleinsäuregestützter Analysemethoden erfolgt die Detektion eines durch ein fluoreszierendes Farbstoffmolekül markierten Analyten (z.B. einer DNA oder RNA) durch Hybridisierung an eine auf dem Träger vorgelegte Matrix aus vielen verschiedenen Oligonukleotiden. Erfahrungsgemäß hat dabei die Dichte der auf dem Träger vorgelegten Oligonukleotide einen maßgeblichen Einfluß auf die Stärke des meßbaren Signales und auf das Verhältnis von Signal zu Hintergrund. Erste Ansätze zur Optimierung der Trägeroberfläche für eine Verbesserung des Detektionsprozesses im Bereich DNA-DNA-Wechselwirkungen sind beschrieben. In einem Ansatz wurde die Chemie aus dem "Starburst"-Dendrimerbreich eingesetzt. Hierbei handelt es sich um stark verzweigte Strukturen, die generationsweise um einen Initationspunktaufgebaut werden. Mit zunehmender Generationszahl nimmt die Zahl der Verzweigungen und damit die Anzahl an Reaktionsorten zu, wodurch es schließlich zu einer Erhöhung der funktionellen Dichte auf der Oberfläche und mittelbar zu einer Verbesserung des Detektionsprozesses (M. Beier, J.D. Hoheisel (1999) Nucleic Acids Res. 27: 1970-1977) kommt.

In einem anderen Ansatz wurden Aminosäuren in die Linkermoleküle integriert, um sowohl für den Synthese, als auch für den Detektionsprozeß (Hybridisierung), die Ladungsdichte auf der Oberfläche pH-abhängig steuern zu können, und so eine hochspezifische Hybridisierung mit dem Analyten zu gewährleisten (M.S. Shchepinov, S.C. Case-Green, E.M. Southern (1997) Nucleic Acid Res. 25: 1155-1161).

Der Erfindung liegt die Aufgabe zugrunde, ein Verfahren bereitzustellen, bei dem die Einführung funktioneller Gruppen "in situ", also während des Funktionalisierungsprozesses, moduliert und dem Verwendungszweck der Oberfläche angepaßt werden kann. Eine weitere Aufgabe besteht darin, im Verfahren geeignete Linkermoleküle nach Art eines Baukastensystems aus einer vorgegebenen Anzahl von chemisch reaktiven Grundbausteinen auswählen und automatisiert kondensieren zu können. Weiterhin sollte das Fortschreiten und das Ergebnis der Synthesereaktion in einem Regelprozeß überwachbar und steuerbar sein.

Außerdem ist die zu lösende Aufgabe die Bereitstellung eines Verfahren, das bei der Synthese oder bei der Immobilisierung von bindungsaktiven Strukturelementen in oder auf einem Reaktionsträger in situ die flexible Einstellung der physikalischen (z.B. Dichte), der physikochemischen (z.B. Polarität), der chemischen (z.B. photochemische Labilität) und/oder der biokompatiblen (z.B. Steuerung molekularer Erkennungsprozesse) Eigenschaften erlaubt, unabhängig von der Vorgabe durch die primäre Funktionalisierung des Reaktionsträgers.

Diese Aufgabe wird gelöst durch den Aufbau eines Verbindungselementes ("Linker") zwischen einer funktionalisierten Festphasenoberfläche und den analytbindenden Strukturelementen (Rezeptoren). Der Linker wird im oder auf dem Reaktionsträger systematisch aus einem oder mehreren gleichen oder verschiedenen Monomeren diverser chemischer Substanzklassen nach Art einer Merrifield-Strategie aufgebaut, die sich im Sinne eines

Baukastenprinzips frei kombinieren lassen. Zur genauen Einstellung der physikalischen, physikochemischen, chemischen und biokompatiblen Eigenschaften der entstehenden aktiven Oberfläche kann eine Steuerung durch einen IST-Wert / ZIEL-Wert Abgleich über eine oder mehrere im oder am Reaktionsträger implementierte Meßzelle(n) erfolgen, die einen frei wählbaren, maßgerechten und reproduzierbaren Aufbau der Linker aus den vorgegebenen Monomereinheiten gestattet.

Im Gegensatz zum Stand der Technik, wo Oberflächeneigenschaften lediglich durch Versuch und Irrtum eingestellt und modifiziert werden können, kann mit Hilfe der Erfindung automatisiert und reproduzierbar eine konfektionierte chemische Oberfläche programmiert aufgebaut werden.

In einem ersten Aspekt umfasst das erfindungsgemäße Verfahren zur Herstellung eines beschichteten Trägers die Schritte
a) Bereitstellen eines Trägers, der an seiner Oberfläche reaktive Gruppen aufweist, und
b) Aufbauen einer funktionalisierten Oberfläche auf dem Träger durch schrittweise Synthese von Linkermolekülen, die funktionelle Gruppen enthalten, aus Synthon-Bausteinen,
wobei der Syntheseprozess der Linkermoleküle überwacht und gegebenenfalls moduliert wird.

Zur Überwachung des Syntheseprozesses werden vorzugsweise für einen oder mehrere physikalisch-chemische Parameter der funktionalisierten Oberfläche ZIEL-Werte vorgegeben, und während der Synthese erfolgt ein Abgleich von gemessenen IST-Werten mit den vorgegebenen ZIEL-Werten des oder der Parameter. Abhängig vom Ergebnis des Abgleichs von IST- und ZIEL-Werten können nachfolgende Syntheseschritte moduliert werden. Die Überwachung kann nach jeweils einem oder mehreren Syntheseschritten erfolgen. Vorzugsweise wird nach jedem Syntheseschritt eine Überwachung durchgeführt. ZIEL-Werte können z.B. vom Experimentator in Abhängigkeit von der Anwendung vorgegeben werden.

Der letzte IST-Wert, der im Idealfall dem jeweils voreingestellten ZIEL-Wert entspricht, kann als IST-Wert für die sich anschließende Kopplung des bindungsaktiven Elements, bzw. Rezeptors an den Linker oder/und als interner Standard für die Qualitätssicherung berücksichtigt werden. Für eine spätere qualitative oder/und quantitative Erfassung des Signals einer chemischen, biochemischen oder biologischen Reaktion an den bindungsaktiven Elementen sind die Oberflächeneigenschaften des Reaktionsträgers in jedem Experiment bekannt und können als Parameter in die Analysen einfließen.

Als Messmethoden zur Überwachung kommen beispielsweise in Betracht: Absorption, Emission, Leitfähigkeit, pH-Wert, NMR, Massenspektrometrie, Radioaktivität, Plasmonenresonanz, Lichtbrechung, Lichtstreuung, Wärmetönung, Elektronenbeugung, Neutronenbeugung oder Ellipsometrie. Die Messung erfolgt in einer Messzelle, die vorzugsweise in den Reaktionsträger selbst oder in die umgebenden fluidischen Elemente integriert ist. Bei fluidisch getrennten Reaktionsräumen innerhalb des Reaktionsträgers erfolgt die Messung vorzugsweise nach Reaktionsräumen getrennt. Bevorzugte ZIEL-Werte bzw. physikalisch-chemische Parameter, die überwacht werden können, sind z.B. die Dichte funktioneller Gruppen auf der Oberfläche, der Abstand funktioneller Gruppen von der Oberfläche, die Oberflächenpolarität, optische, magnetische, elektronische, dielektrische und katalytische Eigen-schaften, die thermische Stabilität, die photochemische und enzymatische Aktivität aber auch räumlich strukturelle Eigenschaften (molekulare Erkennung).

In einem weiteren Aspekt betrifft das erfindungsgemäße Verfahren den Einsatz von Synthon-Bausteinen, die der Modellierung konfektionierter chemischer Oberflächen dienen. Dabei kommen eine Reihe von Bausteinen zum Einsatz, die sich unterschiedlichen chemischen Verbindungsklassen, wie etwa Aminosäuren, Nukleosiden, Glykosiden, zuordnen lassen. Üblicher-weise werden solche Synthon-Bausteine in schutzgruppenmodifizierter Form eingesetzt. Entsprechend ihren chemischen, physikalisch-chemischen und biologischen Eigenschaften können diese Bausteine verschiedene Funktionen zur Konfektionierung der Oberfläche übernehmen: Abstands-halter ("Spacer") wie Alkylreste, dienen der Variation des vertikalen Abstandes zwischen funktionalisiertem Trägermaterial und bindungsaktivem Element. Abstandshalter mit sterisch anspruchsvollen Gruppen können zur Variation der Raumerfüllung, d.h. des horizontalen Abstands zwischen einzelnen Linkermolekülen bindungsaktiven Elementen ("Spacern") ver-wendet werden. Bei Einführung polarer oder geladener Reste in die Abstandshalter (z.B. Verwendung von Oligoethylenglycol anstelle von Alkylresten) kann die Polarität der Oberfläche moduliert werden. Dielek-trische Eigenschaften können beispielsweise durch Einführung von geladenen Gruppen, z.B. Aminosäureresten in den Linker variiert werden.

Die Bausteine können also beispielsweise ausgewählt werden aus Synthonen der Nukleinsäure-, Peptid- oder Kohlenhydratchemie oder/und aus Spacermolekülen.

Die auf dem Träger synthetisierten Linkermoleküle enthalten funktionelle Gruppen, die die Kopplung weiterer Linker-Synthon-Bausteine oder - nach Beendigung der Linkersynthese - von Rezeptoren oder Rezeptorbausteinen ermöglichen. Die funktionellen Gruppen der Linkermoleküle können beispielsweise ausgewählt werden aus -OR, -NR₂, -SR, -PO₃R₂, -CN, -SCN, -COR' und -OCOR', worin R H oder eine Schutzgruppe bedeutet und R' H oder eine Schutzgruppe oder -OR, -NR₂ oder -SR bedeutet. Weiterhin können R und R' für Alkyl, Aryl, Alkenyl und/oder Allylreste und/oder weitere nützliche organische Reste stehen.

Nach beendeter Linkersynthese erfolgt die Kopplung von Rezeptoren, die ebenfalls durch schrittweise Synthese aus Synthese-Bausteinen je nach verwendeter Synthesestrategie, z.B. Peptid-, Oligonukleotid- oder Kohlenhydratsynthese an der Festphase oder durch ortsspezifische und/oder nichtortsspezifische Immobilisierung von kompletten Rezeptoren erfolgen kann.

In noch einem weiteren Aspekt betrifft die vorliegende Erfindung den Einsatz von Dotierungs-Synthonen zur chemischen und physikochemischen, aber auch zur biokompatiblen Modulation der Oberflächeneigenschaften. Hierbei handelt es sich einerseits um verzweigte Synthone, deren Einkopplung zu einer Vervielfachung der Zahl initialer Reaktionsorte führt (Signalamplifikation) und um Blind-Synthone, die zu einer Verringerung der Reaktionsorte führen, da sie selbst keine funktionellen Gruppen tragen (Signalreduktion). Alle Synthone zeichnen sich dadurch aus, dass sie im Sinne einer Merrifield-Synthese einsetzbar sind, d.h., die funktionellen Gruppen der Synthone tragen Schutzgruppen, die unter geeigneten chemischen oder physikalischen Bedingungen selektiv entfernt werden können. Die Einstellung der Oberfächeneigenschaften erfolgt durch Polymerisation, z.B. durch Kondensation einer geeigneten Kombination von Abstandshaltern und Dotierungs-Synthonen. Durch Auswahl von geeigneten Dotierungs-Synthonen wird die Oberflächeneigenschaft moduliert, indem nach jedem Polymerisationsschritt der IST-Wert mit dem ZIEL-Wert verglichen wird, und vom System solange geeignete Synthone oder Mischungen aus geeigneten Synthonen zugeführt werden, bis die Oberfläche die gewünschten Eigenschaften aufweist.

Die erste Addition von Spacer- oder Dotierungs-Synthone kann gefolgt sein von einem Blockierungsschritt, bei dem alle nicht verwendeten funktionellen Gruppen der Oberfläche inaktiviert werden. Im Falle von Glasoberflächen geschieht dies vorzugsweise mit Hexamethyldisilazan, Trüsobutylchlorsilan oder Trimethylchlorsilan. Dann wird die Schutzgruppe von diesem ersten Synthon an der Oberfläche des Reaktionsträgers selektiv entfernt. Anschließend werden - entsprechend dem Ergebnis einer Überwachung der Oberflächeneigenschaften - ein zweiter oder weitere Syntheseschritte mit einer gegebenenfalls modulierten Auswahl von Synthon-Bausteinen hinsichtlich der Anteile an Blind- oder/und Verzweigungs-Synthonen durchgeführt, wobei auch die Länge, die Raumerfüllung oder/und die Polarität der Synthone variiert werden können. Zusätzlich oder alternativ können die Synthonmenge oder/und - konzentrationen oder auch die Reaktionsbedingungen für einzelne oder mehrere Syntheseschritte variiert werden.

Vorzugsweise erfolgt der Syntheseprozess automatisiert und die Einstellung eines Eigenschaftsprofils der funktionalisierten Oberfläche ist programmierbar.

In noch einem weiteren Aspekt betriftt das erfindungsgemäße Verfahren den ortsspezifischen Aufbau der funktionalisierten Oberfläche auf dem Träger, d.h. die funktionalisierte Oberfläche erstreckt sich nicht über den gesamten Träger, sondern nur über vorbestimmte Oberflächenbereiche,
wobei Array-Strukturen erzeugt werden können.

Der ortsspezifische Aufbau der Oberfläche kann durch räumlich oder/und zeitlich begrenzte Belichtung oder durch räumlich oder/und zeitlich begrenzte Fluidzufuhr erfolgen. Dabei können auch mindestens zwei unterschiedlich funktionalisierte Oberflächen auf einem Träger aufgebaut werden. Die Unterschiede zwischen einzelnen Oberflächenbereichen können dabei in der Art der funktionellen Gruppen oder/und in der Dichte der funktionellen Gruppen innerhalb einzelner Bereiche liegen.

Noch ein weiterer Aspekt der vorliegenden Erfindung ist, dass die Kombination unterschiedlicher Syntheseverfahren, z.B. Peptidsynthese bzw. DNA-Synthese oder hybride Syntheseverfahren, unterschiedliche Strategien zur Einstellung der Oberflächeneigenschaften erlaubt. Als eine Variante können auf einem Träger unterschiedliche Oberflächeneigenschaften realisiert werden. Bei Verwendung von ortsspezifischen Syntheseverfahren ist auch die Einstellung von ortsspezifisch unterschiedlichen Oberflächeneigenschaften möglich. Ein solches Verfahren ist beispielsweise die lichtgesteuerte Synthese mit entsprechend photolabilen Schutzgruppen, die über eine geeignete Belichtungsmatrix (siehe z.B. PCT/EP99/06316 und PCT/EP99/06317, auf deren Offenbarung in diesem Zusammenhang ausdrücklich Bezug genommen wird) ortsspezifisch entfernt werden können.

Der Träger kann grundsätzlich beliebig ausgewählt werden, z.B. aus Partikeln, insbesondere magnetischen Partikeln, Mikrotiterplatten und mikrofluidischen Trägern (wie z.B. fluidischen Mikroprozessoren) und kann eine Oberfläche ausgewählt aus Glas, Metallen, Halbmetallen, Metalloxiden oder Kunststoff aufweisen. Besonders bevorzugt sind die in PCT/EP 99/06315 offenbarten Mikropartikel und die in PCT/EP 99/06316 und PCT/EP 99/06317 offenbarten Träger mit planaren bzw. mit Mikrokanälen (Querschnitt z.B. 10 -1000 µm) versehenen Oberflächen. Auf die Offenbarung der genannten Dokumente wird ausdrücklich Bezug genommen.

In einem weiteren Aspekt umfasst das erfindungsgemäße Verfahren das Koppeln von Rezeptoren an die funktionellen Gruppen der Linkermoleküle, wobei das Koppeln der Rezeptoren durch schrittweise Synthese aus Synthon-Bausteinen erfolgen kann. Auch hierbei kann der Syntheseprozess der Rezeptoren überwacht und gegebenenfalls moduliert werden. Rezeptoren sind biologisch oder/und chemisch funktionelle Moleküle, die z.B. ausgewählt werden können aus Nukleinsäuren, Nukleinsäureanaloga, Peptiden, Proteinen und Kohlenhydraten. Alternativ können jedoch auch vorgefertigte Rezeptoren an die Linkermoleküle gekoppelt werden. Auch das Koppeln von Rezeptoren auf dem Träger kann ortsspezifisch erfolgen.

So können z.B. mindestens zwei bezüglich Rezeptorart bzw. Rezeptorsequenz oder/und Rezeptordichte unterschiedliche Rezeptorbereiche aufgebaut werden.

Ein nach dem erfindungsgemäßen Verfahren hergestellter Träger kann z.B. in einem Verfahren zur Bestimmung eines Analyten verwendet werden, gegebenenfalls in einer integrierten Vorrichtung zur Trägersynthese und Analytbestimmung wie in PCT/EP 99/06317 offenbart. Der erfindungsgemäße Träger unterscheidet sich von bekannten Trägern durch seine für den jeweiligen Anwendungszweck optimierte Oberflächenbeschaffenheit und seine hohe Reproduzierbarkeit.

In einem weiteren Aspekt betrifft die Erfindung eine Vorrichtung zur gesteuerten Synthese von Verbindungselementen (Linkern) zwischen reaktiven Gruppen auf der Trägeroberfläche und bindungsaktiven Rezeptoren. Diese Verbindungselemente sind modulare Kondensate aus mehreren gleichen oder verschiedenen Synthon-Bausteinen. Dabei kann die Synthese der Linkermoleküle auf der gesamten Oberfläche des Trägers erfolgen, oder aber ortsspezifisch an ausgewählten Reaktionsorten stattfinden.

Die Vorrichtung zur gesteuerten Synthese von Verbindungselementen kann eine programmierbare Lichtquellenmatrix sein, die eine ortsspezifische lichtabhängige Synthese erlaubt. Eine andere Möglichkeit ist eine Vorrichtung zur fluidischen Synthese, die eine gesteuerte Zuführung von Fluiden, und damit auch der Synthesereagenzien, erlaubt, und dies wahlweise nach Reaktionsbereichen getrennt oder einheitlich.

In einer bevorzugten Ausführungsform umfasst die erfindungsgemäße Vorrichtung zur Herstellung von beschichteten Trägern:
a) mindestens einen Träger, der an seiner Oberfläche reaktive Gruppen aufweist,
b) mehrere Reservoirs, die Lösungen mit Synthon-Bausteinen zur schrittweisen Synthese von Linkermolekülen enthalten,
c) Mittel zur Zufuhr der Synthon-Baustein-Lösungen auf den Träger und zur Ableitung verbrauchter Lösungen vom Träger, z.B. Pumpen und Leitungen oder/und Kapillaren,
d) Mittel zum Überwachen des Syntheseprozesses für die Linkermoleküle, z.B. Meßzellen oder -elemente zur Bestimmung eines oder mehrerer physikalisch-chemischer Parameter,
e) Mittel zum Modulieren des Syntheseprozesses für die Linkermoleküle, z.B. eine entsprechende Software zum Abgleich der IST-Werte für einen oder mehrere Parameter mit vorbestimmten ZIEL-Werten,
f) gegebenenfalls Mittel zum Koppeln von Rezeptoren an Linkermoleküle auf der Trägeroberfläche und
g) gegebenenfalls Mittel zur Durchführung einer Analytbestimmung auf dem Träger, z.B. Mittel zur Zufuhr einer Probe und zur Messung eines durch den Analyten hervorgerufenen Signals.

Die folgenden Figuren und Ausführungsbeispiele sind zur Erläuterung gedacht und sollen den Schutzumfang des erfindungsgemäßen Verfahrens in keiner Weise einschränken.

Es zeigen:
- **Abbildung 1**: ein Beispiel für ein trifunktionelles Linkermolekül, siehe z.B. Burmeister et al. (Tetrahedron Lett. 4995,3667-3668).
- **Abbilung 2**: Beispiele für Fluoreszenzmoleküle auf Tritylbasis und
- **Abbildung 3**: Beispiele für Fluoreszenzmoleküle auf Nitrobenzylbasis.

### Beispiele

Auf einem Reaktionsträger, vorzugsweise einem fluidischen Mikroprozessor aus Glas, werden primäre Alkoholfunktionen eingeführt. Die Beschichtung erfolgt durch einen Prozeß, der aus zwei Teilschritten besteht. Nach einer Aktivierung erfolgt eine mehrstündige Silanisierung durch Bespülen des Reaktionsträgers mit einer Lösung von 1 - 10 % (v:v) Hydroxyalkyltriethoxysilan in einem Alkohol. Für die Durchführbarkeit des erfindungsmäßigen Verfahrens ist eine konstante reproduzierbare Beschichtung wichtig. Dies kann mit verschiedenen Verfahren, wie z.B. mit Ellipsometrie, Glimmentladungsspektroskopie, Konduktometrie oder einer anderen geeigneten Analysemethode, analysiert werden, die sich zum Teil auch für die Detektionsschritte während des Verfahrens eignen. Die entstehenden primären Alkoholfunktionen dienen u.a. als Ausgangspunkte für eine trägergebundene Oligonukleotidsynthese nach der Phosphonat- bzw. Phosphoramiditmethode oder eine Peptidsynthese modifiziert nach Merrifield.

Als Zielvorgabe für eine konfektionierte chemische Oberfläche soll im aufgeführten Beispiel der Abstand zwischen den OH-Funktionen auf der Oberfläche des fluidischen Mikroprozessors und einem nach der Phosphonat- bzw. Phosphoamiditmethode zu synthetisierenden DNA-Oligomer ("bindungsaktives Strukturelement") 50 Atome betragen. Die Stellplatzdichte dieser Oligonukleotide soll auf ca. 10 nmol/cm², die Polarität des Linkers auf eine mittlere Größe eingestellt werden.

Zur Erzeugung des IST-Wertes muß die Funktionalisierungsdichte auf der Oberfläche des fluidischen Mikroprozessors bestimmt werden. Dies geschieht vorzugsweise durch automatisierte Kopplung eines fluoreszierenden dimethoxytrityl- oder eines photoaktiven nitrobenzylgeschützten Monomers aus der Phosphoramiditchemie an freie primäre Alkohole. Prinzipiell kommen für die Einführung der Fluoreszenz verschiedene Ansätze in Frage:

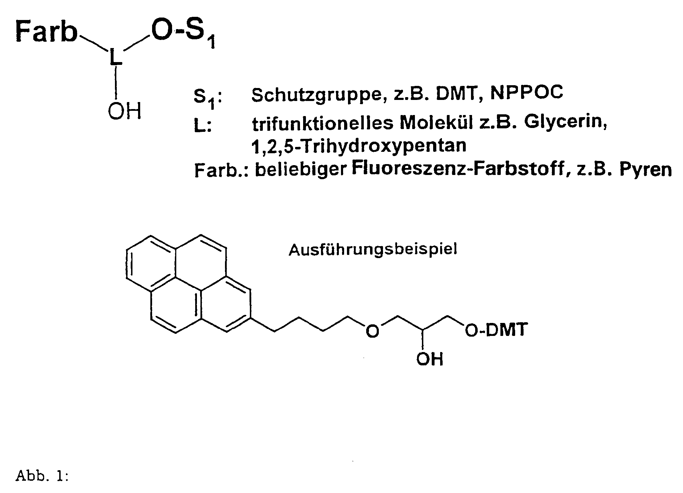

### Fluoreszenz auf Linkerbasis (Abb. 1):

Bei dieser Substanzklasse werden trifunktionale Linkermoleküle verwendet, an deren eine Funktionalität ein Farbstoff (Farb) und an deren andere eine Schutzgruppe (S₁) gekoppelt wurden.

Die Fluoreszenzfarbstoffe bleiben in diesem Fall während des gesamten Prozesses im System. Die eigentliche Oligonukleotidsynthese erfolgt nach Abspaltung der Schutzgruppe S₁. Im Ausführungsbeispiel 3-O-(4,4'-Dimethoxytrityl)-1-O-(1-pyrenyl)-butyl-glycerin (Burmeister et al., supra) wurde Pyren als Farbstoff und Dimethoxytrityl (DMT) als Schutzgruppe S₁ genutzt.

Um Nebenreaktionen zurückzudrängen, ist bei Verwendung von Photoschutzgruppen für Position S₁ darauf zu achten, dass sich die Anregungswellenlängen für die Abspaltung der Schutzgruppe und die Anregung der Fluoreszenz nicht überschneiden.

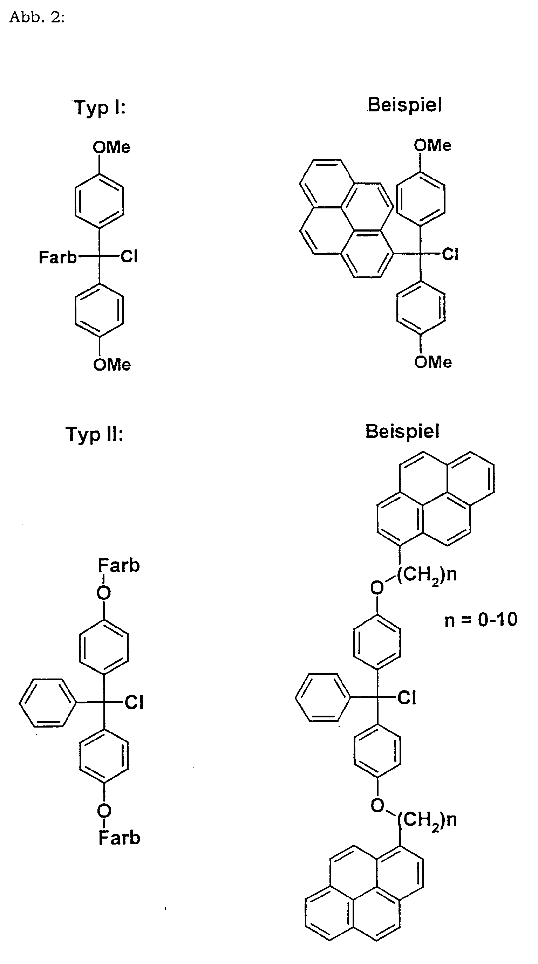

### Fluoreszenz auf Tritylschutzgruppenbasis (Abb. 2):

Einführung der Fluoreszenz erfolgt einerseits durch Substitution der Phenylgruppe im 4,4'-Dimethoxytriphenylmethylchlorid (DMT-CI) durch einen geeigneten Farbstoff, z.B. Pyren (Typ I) andererseits durch Substitution eines oder beider Methylgruppen der Methoxyfunktionalitäten, z.B. Pyrenbutanol (Typ II). Entscheidend ist die Fähigkeit, das bei der Abspaltung der Schutzgruppe entstehende Kation zu stabilisieren, nicht verloren geht.

Vorteil dieser Methode ist es, dass sich die fluoreszierenden säurelabilen Tritylschutzgruppen ohne weiteres in die Standard-Oligonukleotidsynthese integrieren lassen.

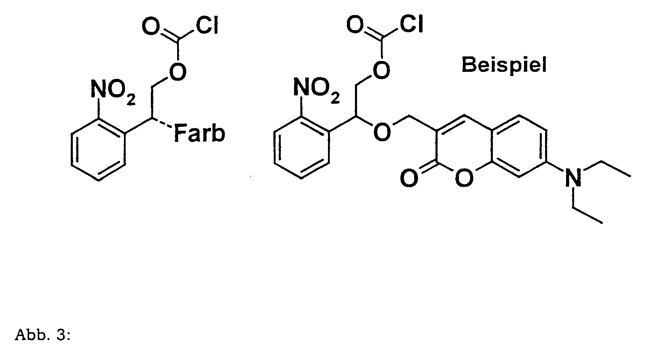

### Fluoreszenz auf Nitrobenzylschutzgruppenbasis (Abb. 3):

Neben den säurelabilen DMT-Schutzgruppen werden auch Strategien verwendet, die auf der Abspaltung photoaktiver Schutzgruppen basieren. In der Regel handelt es sich dabei um Nitrobenzylderivate. Durch gezielte Inkorporation einer geeigneten fluorophoren Gruppe, wie etwa einer 7-(Dimethylamino)-cumarin-Gruppierung, lassen sich diese fluoreszierenden photolabilen Schutzgruppen in die Oligonukleotidsynthese integrieren.

### Fluoreszenz auf Basis von Nanopartikeln:

Als weitere Quelle für Fluoreszenz dienen Goldpartikel. Werden diese reversibel immobilisierten Nanoteilchen unter einem bestimmten Winkel bestrahlt, treten Light Scattering Effekte auf.

Das Ausmaß der auftretenden Fluoreszenz gibt ein indirektes Maß für die Zahl der zugänglichen primären OH-Funktionen. Der gefundene Wert dient als IST-Wert für die Stellplatzdichte des gewünschten Linkers. Dieser IST-Wert wird mit dem Zielwert verglichen. Je nachdem, ob die Vorgabe über- oder unterschritten ist, wird im nächsten Kopplungsschritt ein geeignetes Monomer oder ein Gemisch aus geeigneten Monomeren appliziert (Eine Palette an Monomeren ist im System bevorratet. Die Reaktivitätsdaten der Monomere sind in einer elektronischen Datenbank enthalten.), vorzugsweise ein Gemisch aus einem Blindmonomer, z.B. Methoxy-[(2-cyanoethyl)-(N,N-diisopropyl)]phosphoramidit und einem fluoreszierenden Verzweigungsmonomer.

Wiederum wird eine Fluoreszenzmessung zur Bestimmung des neuen IST-Wertes herangezogen. Dieser Schritt wird sukzessive wiederholt, bis die gewünschte Funktionalisierungsdichte erreicht ist. Anschließend erfolgt die Einstellung des Abstandes zwischen der Trägeroberlfäche und dem zu synthetisierenden DNA-Oligonukleotid durch die schrittweise Einkopplung von vorzugsweise kommerziell erhältlichen Spaceramiditen. Ist ein Abstand von z.B. 40 - 60, vorzugsweise ca. 50 Atomen, zur Trägeroberfläche realisiert, kann mit der automatisierten Synthese des gewünschten DNA-Oligonukleotids begonnen werden. Anstelle der Dimethoxytrityl geschützten Monomere können auch photolabile, z.B. mit 2-Nitrobenzyloxycarbonyl oder 2-Nitroethyloxycarbonyl geschützte Monomere eingesetzt werden. Bei Verwendung einer programmierbaren Lichtquellenmatrix zur Belichtung auf dem fluidischen Mikroprozessor, können so ortsspezifisch verschiedene Linkermoleküle aufgebaut werden. Vor allem im Falle der Nanopartikel stellt die Ellipsometrie eine weitere Methode zur Generierung von IST-Werten dar.

Alle aufgeführten Varianten, die auf Fluoreszenz beruhen, vereinigen in sich den Vorteil, dass sie die Justage des optischen Aufbaus in einem Trägersystem gemäß PCT/EP99/06317 erheblich vereinfachen, da die Fokussierung auf diskrete Schichten wesentlich genauer ist, als die Fokussierung auf eine diffuse fluoreszierende Lösung.

Als weitere Methode kann die UV/VIS-Absorption des Tritylkations (λ = 498 nm) des gekoppelten Amidits (vorzugsweise 3-Dimethoxytrityloxypropyl-1-[(2-cyanoethyl)-(N,N-diisopropyl)]-phosphoramidit),nacherfolgter Entschützungsreaktion zur Ermittlung des IST-Werts herangezogen werden. Sie ist ein direktes Maß für die Zahl der zugänglichen primären OH-Funktionen. Der gefundene Wert dient als IST-Wert für die Stellplatzdichte des gewünschten Linkers. Dieser IST-Wert wird mit dem Zielwert (10 nmol/cm²) verglichen. Je nachdem ob die Vorgabe über- oder unterschritten ist, wird im nächsten Kopplungsschritt ein geeignetes Monomer oder ein Gemisch aus geeigneten Monomeren (eine Palette an Monomeren ist im System bevorratet, die Reaktivitätsdaten der Monomere sind in einer elektronischen Datenbank enthalten), vorzugsweise Methoxy-[(2-cyanoethyl)-(N,N-diisopropyl)]phosphoramidit ("Blindmonomer") und [4,4'-Dimethoxytrityloxyl-1,1-bis-ethyl-[(2-cyanoethyl)-(N,N-diisopropyl)]-phosphoramidit ("Verzweigungsmonomer") appliziert. Wiederum wird die Absorptionsmessung der Dimethoxytritylkationen zur Bestimmung des neuen IST-Wertes herangezogen. Dieser Schritt wird sukzessive wiederholt, bis die gewünschte Funktionalisierungsdichte erreicht ist. Anschließend erfolgt die Einstellung des Abstandes zwischen der Trägeroberfläche und dem zu synthetisierenden DNA-Oligonukleotid durch die schrittweise Einkopplung von Spaceramiditen, vorzugsweise 3-Dimethoxytrityloxypropyl-1-[(2-cyanoethyl)-(N,N-diisopropyl)]phosphoramidit bzw. zur Erhöhung der Polarität 9-Dimethoxytrityloxy-triethylenglycol, 1-[(2-cyanoethyl)-(N,N-düsopropyl)]phosphoramidit. Ist ein Abstand von 50 Atomen zur Trägeroberfläche realisiert, kann mit der automatisierten Synthese des gewünschen DNA-Oligonukleotids begonnen werden.

Anstelle der Dimethoxytrityl-geschützten Monomere können auch photolabile, mit 2-Nitrobenyloxycarbonyl oder 2-Nitroethyloxycarbonyl geschützte Monomere eingesetzt werden. Bei Verwendung einer programmierbaren Lichtquellenmatrix zur Belichtung auf dem fluidischen Mikroprozessor, können so ortsspezifisch verschiedene Linkermoleküle aufgebaut werden.

Als eine alternative Ausführung wird der Linker gemäß oben beschriebenem Verfahren bereitgestellt. Anschließend dienen die Anlagerungsstellen in Form entschützter funktioneller Gruppen der Immobilisierung von fertigen Polymersonden. Dabei kann eine ortsaufgelöste Entschützung die entsprechend ortsaufgelöste Immobilisierung gegebenenfalls unterschiedlicher Sonden ermöglichen.

In einer weiteren Ausführung wird die Schutzgruppentechnik für die ortsaufgelöst unterschiedliche Dotierung der Oberfläche verwendet, so daß in unterschiedlichen Reaktionsbereichen unterschiedliche Konzentrationen bzw. Dichten gleicher oder verschiedener Polymersonden entstehen.

Dabei kann wiederum mit automatisierter Erfassung und Einstellung eine einheitliche definierte Dichte an Spacer-Molekülen bereitgestellt werden. Im Anschluß daran kann bei Verwendung einer ortsaufgelösten Entfernung der Schutzgruppe eine Fraktion der Reaktionsbereiche aktiviert und eine Polymersondensynthese begonnen werden. Für die Dichte der in diesen Reaktionsbereichen enstehenden Sonden kann das geeignete Gemisch von verlängerbaren, nicht verlängerbaren oder verzweigten Synthonen wie beschrieben zugegeben werden. Polymersonden gleicher Sequenz, aber unterschiedlicher Dichte können in der Folge synthetisiert werden, indem nun andere Reaktionsbereiche aktiviert und mit einem Gemisch in Kontakt gebracht werden, das die gleiche Art von Bausteinen enthält, aber eine andere Mischung der verlängerbaren, nicht-verlängerbaren oder verzweigten Synthone. Auf entsprechende Weise können auch Reaktionsbereiche mit unterschiedlichen Konzentrationen an verschiedenen Polymersonden hergestellt werden. Alternativ kann die Dichte der Sonden so eingestellt werden, indem bei bekannter Halbwertszeit T_{1/2} einer Schutzgruppe die Exposition zum entschützenden Einfluß, z.B. einer Lichtquelle, so gewählt wird, daß nur ein definierter Anteil der Schutzgruppen entfernt wird und somit die Dichte der anschließend hergestellten Polymersonden auf dem Reaktionsbereich eingestellt wird. Somit können bei ortsaufgelöster Entschützung, z.B. lichtabhängier Entschützung, Polymersonden gleicher oder verschiedener Sequenz an unterschiedlichen Orten mit unterschiedlicher Dichte synthetisiert werden. Dies kann unter anderem für quantitative Bestimmung des Meßsignals ausgenutzt werden, z.B. indem die Dichte der Polymersonden die Anzahl der gebundenen Analytmoleküle und somit die Signalstärke beeinflußt.

## Patentansprüche

1. Verfahren zur Herstellung eines beschichteten Trägers, umfassend die Schritte,
a) Bereitstellen eines Trägers, der an seiner Oberfläche reaktive Gruppen aufweist, und
b) Aufbauen einer funktionalisierten Oberfläche auf dem Träger durch schrittweise Synthese von Linkermolekülen, die funktionelle Gruppen enthalten, aus Synthon-Bausteinen,
**dadurch gekennzeichnet,**
**dass** der Syntheseprozess der Linkermoleküle überwacht und gegebenenfalls moduliert wird.

2. Verfahren nach Anspruch 1,
**dadurch gekennzeichnet,**
**dass** für einen oder mehrere physikalisch-chemische Parameter der funktionalisierten Oberfläche ZIEL-Werte vorgegeben werden und dass während der Synthese ein Abgleich von gemessenen IST-Werten mit den vorgegebenen ZIEL-Werten des oder der Parameter erfolgt.

3. Verfahren nach Anspruch 2,
**dadurch gekennzeichnet,**
**dass** abhängig vom Ergebnis des Abgleichs von IST- und ZIEL-Werten nachfolgende Syntheseschritte moduliert werden.

4. Verfahren nach einem der Ansprüche 1-3,
**dadurch gekennzeichnet,**
**dass** eine Überwachung nach jedem Syntheseschritt durchgeführt wird.

5. Verfahren nach einem der Ansprüche 1-4,
**dadurch gekennzeichnet,**
**dass** die physikalisch-chemischen Parameter ausgewählt werden aus der Dichte funktioneller Gruppen auf der Oberfläche, dem Abstand funktioneller Gruppen von der Oberfläche, der Oberflächenpolarität, optischen, magnetischen, elektronischen, dielektrischen und katalytischen Eigenschaften, der thermischen Stabilität, der photochemischen und enzymatischen Aktivität und räumlich strukturellen Eigenschaften.

6. Verfahren nach einem der Ansprüche 1-5,
**dadurch gekennzeichnet,**
**dass** die Überwachung durch eine oder mehrere im oder am Träger angebrachte Messzellen erfolgt.

7. Verfahren nach einem der Ansprüche 1-6,
**dadurch gekennzeichnet,**
**dass** die Überwachung durch Bestimmung von Absorption, Emission, Leitfähigkeit, pH-Wert, NMR, Massenspektrometrie, Radioaktivität, Plasmonenresonanz, Lichtbrechung, Lichtstreuung, Wärmetönung, Elektronenbeugung, Neutronenbeugung oder Ellipsometrie erfolgt.

8. Verfahren nach einem der vorhergehenden Ansprüche,
**dadurch gekennzeichnet,**
**dass** der Syntheseprozess automatisiert erfolgt.

9. Verfahren nach einem der vorhergehenden Ansprüche,
**dadurch gekennzeichnet,**
**dass** die Einstellung eines Eigenschaftsprofils der funktionalisierten Oberfläche programmierbar ist.

10. Verfahren nach einem der vorhergehenden Ansprüche,
**dadurch gekennzeichnet,**
**dass** eine Modulation des Syntheseprozesses durch Modulation der Auswahl von Synthon-Bausteinen für einen oder mehrere Syntheseschritte erfolgt.

11. Verfahren nach Anspruch 10,
**dadurch gekennzeichnet,**
**dass** die Synthonmenge oder/und -konzentration für einen oder mehrere Syntheseschritte variiert wird.

12. Verfahren nach Anspruch 10 oder 11,
**dadurch gekennzeichnet,**
**dass** der Anteil an Blind- oder/und Verzweigungssynthonen für einen oder mehrere Syntheseschritte variiert wird.

13. Verfahren nach einem der Ansprüche 10-12,
**dadurch gekennzeichnet,**
**dass** die Länge, die Raumerfüllung oder/und die Polarität von Synthonen für einen oder mehrere Syntheseschritte variiert wird.

14. Verfahren nach einem Ansprüche 10-13,
**dadurch gekennzeichnet,**
**dass** die Reaktionsbedingungen für einen oder mehrere Syntheseschritte variiert werden.

15. Verfahren nach einem der vorhergehenden Ansprüche,
**dadurch gekennzeichnet,**
**dass** der Aufbau der funktionalisierten Oberfläche auf dem Träger ortsspezifisch erfolgt.

16. Verfahren nach Anspruch 15,
**dadurch gekennzeichnet,**
**dass** der ortsspezifische Aufbau der Oberfläche durch räumlich oder/und zeitlich begrenzte Belichtung erfolgt.

17. Verfahren nach Anspruch 15 oder 16,
**dadurch gekennzeichnet,**
**dass** der ortsspezifische Aufbau der Oberfläche durch räumlich oder/und zeitlich begrenzte Fluidzufuhr erfolgt.

18. Verfahren nach einem der Ansprüche 15-17,
**dadurch gekennzeichnet,**
**dass** mindestens zwei unterschiedlich funktionalisierte Oberflächenbereiche auf einem Träger aufgebaut werden.

19. Verfahren nach Anspruch 18,
**dadurch gekennzeichnet,**
**dass** auf dem Träger Oberflächen aufgebaut werden, die sich bezüglich der Art oder/und der Dichte der funktionellen Gruppen innerhalb einzelner Bereiche unterscheiden.

20. Verfahren nach einem der vorhergehenden Ansprüche,
**dadurch gekennzeichnet,**
**dass** die Bausteine ausgewählt werden aus Synthonen der Nukleinsäure-, Peptid- oder Kohlenhydratchemie.

21. Verfahren nach einem der vorhergehenden Ansprüche,
**dadurch gekennzeichnet,**
**dass** die Bausteine ausgewählt werden aus Spacermolekülen.

22. Verfahren nach einem der vorhergehenden Ansprüche,
**dadurch gekennzeichnet,**
**dass** der Träger eine Oberfläche ausgewählt aus Glas, Metallen, Halbmetallen, Metalloxiden oder Kunststoff aufweist.

23. Verfahren nach einem der vorhergehenden Ansprüche,
**dadurch gekennzeichnet,**
**dass** der Träger ausgewählt wird aus Partikeln, insbesondere magnetischen Partikeln, Mikrotiterplatten und mikrofluidischen Trägern.

24. Verfahren nach einem der vorhergehenden Ansprüche,
**dadurch gekennzeichnet,**
**dass** die funktionellen Gruppen der Linkermoleküle ausgewählt werden aus -OR, -NR₂, -SR, -PO₃R₂, -CN, -SCN, -COR' und -OCOR', worin R H oder eine Schutzgruppe bedeutet und R' H oder eine Schutzgruppe oder -OR, -NR₂ oder -SR bedeutet.

25. Verfahren nach einem der vorhergehenden Ansprüche,
weiterhin umfassend
das Koppeln von Rezeptoren an die funktionellen Gruppen der Linkermoleküle.

26. Verfahren nach Anspruch 25,
**dadurch gekennzeichnet,**
**dass** das Koppeln durch schrittweise Synthese von Rezeptoren aus Synthon-Bausteinen erfolgt.

27. Verfahren nach Anspruch 26,
**dadurch gekennzeichnet,**
**dass** der Syntheseprozess der Rezeptoren überwacht und gegebenenfalls moduliert wird.

28. Verfahren nach einem der Ansprüche 24-27,
**dadurch gekennzeichnet,**
**dass** die Rezeptoren ausgewählt werden aus Nukleinsäuren, Nukleinsäureanaloga, Peptiden, Proteinen und Kohlenhydraten.

29. Verfahren nach einem der Ansprüche 24 bis 28,
**dadurch gekennzeichnet,**
**dass** das Koppeln von Rezeptoren auf den Träger ortsspezifisch erfolgt.

30. Verfahren nach Anspruch 29,
**dadurch gekennzeichnet,**
**dass** auf dem Träger mindestens zwei bezüglich der Art oder/und der Dichte der Rezeptoren unterschiedliche Rezeptorbereiche aufgebaut werden.

31. Verwendung eines nach einem der Ansprüche 1-30 hergestellten Trägers in einem Verfahren zur Bestimmung eines Analyten.

32. Vorrichtung zur Herstellung von beschichteten Trägern, umfassend:
a) mindestens einen Träger, der an seiner Oberfläche reaktive Gruppen aufweist,
b) mehrere Reservoirs, die Lösungen mit Synthon-Bausteinen zur schrittweisen Synthese von Linkermolekülen enthalten,
c) Mittel zur Zufuhr der Synthon-Baustein-Lösungen auf den Träger und zur Ableitung verbrauchter Lösungen vom Träger,
d) Mittel zum Überwachen des Syntheseprozesses für die Linkermoleküle und
e) Mittel zum Modulieren des Syntheseprozesses für die Linkermoleküle.

33. Vorrichtung nach Anspruch 32,
**weiterhin umfassend**
f) Mittel zum Koppeln von Rezeptoren an Linkermoleküle auf der Trägeroberfläche.

34. Vorrichtung nach Anspruch 32 oder 33, **weiterhin umfassend**
g) Mittel zur Durchführung einer Analytbestimmung auf dem Träger.

## Claims

1. Method for producing a coated support comprising the steps
a) providing a support which has reactive groups on its surface, and
b) synthesizing a functionalized surface on the support by stepwise synthesis of linker molecules which contain functional groups from synthon building blocks,
**characterized in that**,
the process of synthesizing the linker molecules is monitored and optionally modulated.

2. Method as claimed in claim 1,
**characterized in that**
TARGET values are specified for one or more physicochemical parameters of the functionalized surface and **in that** during the synthesis measured ACTUAL values are compared with the specified TARGET values of the parameter(s).

3. Method as claimed in claim 2,
**characterized in that**
subsequent synthesis steps are modulated depending on the result of the comparison of ACTUAL and TARGET values.

4. Method as claimed in one of the claims 1-3,
**characterized in that**
a monitoring is carried out after each synthesis step.

5. Method as claimed in one of the claims 1-4,
**characterized in that**
the physicochemical parameters are selected from the density of functional groups on the surface, the distance of functional groups from the surface, the surface polarity, optical, magnetic, electronic, dielectric and catalytic properties, the thermal stability, the photochemical and enzymatic activity and spatially structural properties.

6. Method as claimed in one of the claims 1-5,
**characterized in that**
the monitoring is carried out by one or more measuring cells installed in or on the support.

7. Method as claimed in one of claims 1-6,
**characterized in that**
the monitoring takes place by determining absorption, emission, conductivity, pH, NMR, mass spectrometry, radioactivity, plasmon resonance, light refraction, light scattering, heat evolution, electron diffraction, neutron diffraction or ellipsometry.

8. Method as claimed in one of the previous claims,
**characterized in that**
the synthesis process is automated.

9. Method as claimed in one of the previous claims,
**characterized in that**
the adjustment of a property profile of the functionalized surface can be programmed.

10. Method as claimed in one of the previous claims,
**characterized in that**
modulation of the synthesis process takes place by modulation of the selection of synthon building blocks for one or more synthesis steps.

11. Method as claimed in claim 10,
**characterized in that**
the amount or/and concentration of synthons is varied for one or more synthesis steps.

12. Method as claimed in claim 10 or 11,
**characterized in that**
the proportion of blind or/and branching synthons is varied for one or more synthesis steps.

13. Method as claimed in one of the claims 10-12,
**characterized in that**
the length, the bulk or/and the polarity of synthons is varied for one or more synthesis steps.

14. Method as claimed in one of the claims 10-13,
**characterized in that**
the reaction conditions are varied for one or more synthesis steps.

15. Method as claimed in one of the previous claims,
**characterized in that**
the functionalized surface on the support is synthesized in a site-specific manner.

16. Method as claimed in claim 15,
**characterized in that**
the site-specific synthesis of the surface takes place by spatially or/and temporally limited exposure to light.

17. Method as claimed in claim 15 or 16,
**characterized in that**
the site-specific synthesis of the surface takes place by spatially or/and temporally limited fluid supply.

18. Method as claimed in one of the claims 15-17,
**characterized in that**
at least two differently functionalized surface regions are synthesized on one support.

19. Method as claimed in claim 18,
**characterized in that**
surfaces are synthesized on the support which differ with regard to the nature or/and the density of the functional groups within individual regions.

20. Method as claimed in one of the previous claims,
**characterized in that**
the building blocks are selected from synthons of nucleic acid, peptide or carbohydrate chemistry.

21. Method as claimed in one of the previous claims,
**characterized in that**
the building blocks are selected from spacer molecules.

22. Method as claimed in one of the previous claims,
**characterized in that**
the support has a surface selected from glass, metals, semimetals metalloids, metal oxides or plastics.

23. Method as claimed in one of the previous claims,
**characterized in that**
the support is selected from particles, in particular magnetic particles, microtitre plates and microfluidic supports.

24. Method as claimed in one of the previous claims,
**characterized in that**
the functional groups of the linker molecules are selected from -OR, -NR₂, -SR, -PO₃R₂, -CN, -SCN, -COR' and -OCOR' in which R denotes H or a protective group, and R' denotes H or a protective group or -OR, -NR₂ or -SR.

25. Method as claimed in one of the previous claims,
**additionally comprising**
coupling of receptors to the functional groups of the linker molecules.

26. Method as claimed in claim 25,
**characterized in that**
the coupling takes place by stepwise synthesis of receptors from synthon building blocks.

27. Method as claimed in claim 26,
**characterized in that**
the process for synthesizing the receptors is monitored and optionally modulated.

28. Method as claimed in one of the claims 24-27,
**characterized in that**
the receptors are selected from nucleic acids, nucleic acid analogues, peptides, proteins and carbohydrates.

29. Method as claimed in one of the claims 24 to 28,
**characterized in that**
receptors are coupled site-specifically to the support.

30. Method as claimed in claim 29,
**characterized in that**
at least two receptor regions which differ with regard to the nature or/and the density of the receptors are synthesized on the support.

31. Use of a support produced as claimed in one of the claims 1-30 in a method for determining an analyte.

32. Device for producing coated supports comprising:
a) at least one support which has reactive groups on its surface,
b) a plurality of reservoirs which contain solutions containing synthon building blocks for the stepwise synthesis of linker molecules,
c) means for supplying the synthon building block solutions to the support and for conducting away used solutions from the support,
d) means for monitoring the process for synthesizing the linker molecules, and
e) means for modulating the process for synthesizing the linker molecules.

33. Device as claimed in claim 32, **additionally comprising**
f) means for coupling receptors to linker molecules on the support surface.

34. Device as claimed in claim 32 or 33, **additionally comprising**
g) means for carrying out an analyte determination on the support.

## Revendications

1. Procédé de préparation d'un support revêtu, comprenant les étapes de
a) préparation d'un support présentant des groupes réactifs sur sa surface, et
b) constitution d'une surface fonctionnalisée sur le support par synthèse par étapes de molécules de liaison contenant des groupes fonctionnels, à partir d'éléments structuraux synthons,
**caractérisé en ce que** le procédé de synthèse des molécules de liaison est contrôlé et éventuellement modulé.

2. Procédé selon la revendication 1, **caractérisé en ce que** des valeurs cibles pour un ou plusieurs paramètres physico-chimiques de la surface fonctionnalisée sont prédéfinies et **en ce que**, pendant la synthèse, les valeurs réelles mesurées sont comparées aux valeurs cibles prédéfinies du ou des paramètres.

3. Procédé selon la revendication 2, **caractérisé en ce que**, en fonction du résultat de la comparaison des valeurs réelles et cibles, les étapes de synthèse suivantes sont modulées.

4. Procédé selon l'une des revendications 1-3, **caractérisé en ce qu'**un contrôle est effectué après chaque étape de synthèse.

5. Procédé selon l'une des revendications 1-4, **caractérisé en ce que** les paramètres physico-chimiques sont choisis parmi la densité de groupes fonctionnels sur la surface, la distance entre les groupes fonctionnels et la surface, la polarité de la surface, des propriétés optiques, magnétiques, électroniques, diélectriques et catalytiques, la stabilité thermique, l'activité photochimique et enzymatique et les propriétés de structure spatiale.

6. Procédé selon l'une des revendications 1-5, **caractérisé en ce que** le contrôle est réalisé par une ou plusieurs cellules de mesure disposées dans ou sur le support.

7. Procédé selon l'une des revendications 1-6, **caractérisé en ce que** le contrôle s'effectue par détermination de l'absorption, de l'émission, de la conductivité, du pH, par RMN, par spectrométrie de masse, par mesure de la radioactivité, de la résonance plasma, de la réfraction, de la dispersion de la lumière, de la tonalité thermique, de la diffraction d'électrons, de la diffraction de neutrons ou par ellipsométrie.

8. Procédé selon l'une des revendications précédentes, **caractérisé en ce que** le procédé de synthèse s'effectue de manière automatisée.

9. Procédé selon l'une des revendications précédentes, **caractérisé en ce que** l'établissement d'un profil de propriétés de la surface fonctionnalisée est programmable.

10. Procédé selon l'une des revendications précédentes, **caractérisé en ce qu'**une modulation du procédé de synthèse s'effectue par modulation du choix d'éléments structuraux synthons pour une ou plusieurs étapes de synthèse.

11. Procédé selon la revendication 10, **caractérisé en ce que** l'on fait varier la quantité et/ou la concentration de synthons pour une ou plusieurs étapes de synthèse.

12. Procédé selon la revendication 10 ou 11, **caractérisé en ce que** l'on fait varier la fraction de synthons aveugles et/ou de synthons de ramification pour une ou plusieurs étapes de réaction.

13. Procédé selon l'une des revendications 10-12, **caractérisé en ce que** l'on fait varier la longueur, l'encombrement stérique et/ou la polarité de synthons pour une ou plusieurs étapes de synthèse.

14. Procédé selon l'une des revendications 10-13, **caractérisé en ce que** l'on fait varier les conditions réactionnelles pour une ou plusieurs étapes de synthèse.

15. Procédé selon l'une des revendications précédentes, **caractérisé en ce que** la constitution de la surface fonctionnalisée sur le support s'effectue de manière localisée.

16. Procédé selon la revendication 15, **caractérisé en ce que** la constitution de la surface localisée s'effectue par exposition à la lumière limitée dans l'espace et/ou le temps.

17. Procédé selon la revendication 15 ou 16, **caractérisé en ce que** la constitution de la surface localisée s'effectue par amenée de fluide limitée dans l'espace et/ou le temps.

18. Procédé selon l'une des revendications 15-17, **caractérisé en ce qu'**au moins deux zones de surface fonctionnalisées de manière différente sont constituées sur un support.

19. Procédé selon la revendication 18, **caractérisé en ce que** l'on constitue sur le support des surfaces qui se distinguent à l'intérieur de différentes zones par le type et/ou la densité des groupes fonctionnels.

20. Procédé selon l'une des revendications précédentes, **caractérisé en ce que** les éléments structuraux sont choisis parmi des synthons de la chimie des acides nucléiques, des peptides ou des hydrates de carbone.

21. Procédé selon l'une des revendications précédentes, **caractérisé en ce que** les éléments structuraux sont choisis parmi des molécules d'espaceurs.

22. Procédé selon l'une des revendications précédentes, **caractérisé en ce que** le support présente une surface choisie parmi le verre, les métaux, les semi-métaux, les oxydes métalliques ou les matières plastiques.

23. Procédé selon l'une des revendications précédentes, **caractérisé en ce que** le support est choisi parmi des particules, en particulier des particules magnétiques, des plaques de microtitrage et des supports microfluidiques.

24. Procédé selon l'une des revendications précédentes, **caractérisé en ce que** les groupes fonctionnels des molécules de liaison sont choisis parmi -OR, -NR₂, -SR, -PO₃R₂, -CN, -SCN, -COR' et -OCOR', où R représente H ou un groupe protecteur et R' représente H ou un groupe protecteur ou -OR, -NR₂ ou -SR.

25. Procédé selon l'une des revendications précédentes, comprenant en outre le couplage de récepteurs aux groupes fonctionnels des molécules de liaison.

26. Procédé selon la revendication 25, **caractérisé en ce que** le couplage s'effectue par synthèse par étapes de récepteurs à partir d'éléments structuraux de type synthons.

27. Procédé selon la revendication 26, **caractérisé en ce que** le procédé de synthèse des récepteurs est contrôlé et éventuellement modulé.

28. Procédé selon l'une des revendications 24-27, **caractérisé en ce que** les récepteurs sont choisis parmi des acides nucléiques, des analogues d'acides nucléiques, des peptides, des protéines et des hydrates de carbone.

29. Procédé selon l'une des revendications 24 à 28, **caractérisé en ce que** le couplage de récepteurs sur le support s'effectue de manière localisée.

30. Procédé selon la revendication 29, **caractérisé en ce qu'**au moins deux zones de récepteurs différentes en ce qui concerne le type et/ou la densité des récepteurs sont constituées sur le support.

31. Utilisation d'un support préparé selon l'une des revendications 1-30 dans un procédé de détermination d'un analyte.

32. Dispositif pour la préparation de supports revêtus, comprenant:
a) au moins un support présentant des groupes réactifs sur sa surface,
b) plusieurs réservoirs contenant des solutions de motifs structuraux synthons pour la synthèse par étapes de molécules de liaison,
c) des moyens pour amener les solutions d'éléments structuraux synthons sur le support et pour éliminer les solutions usées du support,
d) des moyens pour contrôler le procédé de synthèse de molécules de liaison, et
e) des moyens pour moduler le procédé de synthèse de molécules de liaison.

33. Dispositif selon la revendication 32, comprenant en outre
f) des moyens pour coupler des récepteurs aux molécules de liaison sur la surface du support.

34. Dispositif selon la revendication 32 ou 33, comprenant en outre
g) des moyens pour effectuer une détermination d'analyte sur le support.
